# EUROPÄISCHE PATENTSCHRIFT

(11) **EP 3 865 065 B1**
(45) Veröffentlichungstag und Bekanntmachung des Hinweises auf die Patenterteilung: **03.06.2026**
(21) Anmeldenummer: 21156166.7
(22) Anmeldetag: 10.02.2021
(51) Int. Cl.: A61B 5/318, G06N 3/08, G16H 50/20, A61B 5/00, G06N 3/0499, G06N 3/09

(54) **VERFAHREN ZUR VORHERSAGE UND LOKALISIERUNG STRUKTURELLER VERÄNDERUNGEN IM MYOKARDGEWEBE**
METHOD FOR PREDICTING AND LOCATING STRUCTURAL CHANGES IN MYOCARDIAL TISSUE
PROCÉDÉ DE PRÉDICTION ET DE LOCALISATION DES CHANGEMENTS DE STRUCTURE DU TISSU MYOCARDIAL

(30) Priorität: 14.02.2020 DE 102020103943
(43) Veröffentlichungstag der Anmeldung: 18.08.2021
(73) Patentinhaber: Technische Hochschule Mittelhessen, Körperschaft des öffentlichen Rechts, 35390 Giessen (DE); Justus-Liebig-Universität Gießen, Körperschaft des öffentlichen Rechts, 35390 Gießen (DE)
(72) Erfinder: Grün, Dimitri, 61392 Bad Nauheim (DE); Guckert, Michael, 61231 Bad Nauheim (DE); Gumpfer, Nils, 34212 Melsungen (DE); Hannig, Jennifer, 61118 Bad Vilbel (DE); Hurka, Sabine, 35586 Wetzlar (DE); Keller, Till, 60528 Frankfurt am Main (DE)
(74) Vertreter: Stumpf, Peter

(56) Entgegenhaltungen:
- WO-A1-2017/072250
- CN-A- 110 226 920
- CN-A- 110 495 877
- US-A1- 2016 135 703
- US-A1- 2017 273 586
- US-A1- 2019 090 774
- US-A1- 2019 298 195
- ANONYMOUS JOS� T ET AL: "Correspondence Between the 17-Segment Model and Coronary Arterial Anatomy Using Contrast-Enhanced Cardiac Magnetic Resonance Imaging | JACC: Cardiovascular Imaging", 1 May 2008 (2008-05-01), XP093261007, Retrieved from the Internet <URL:https://www.jacc.org/doi/10.1016/j.jcmg.2008.01.014?utm_source=chatgpt.com>

## Beschreibung

Die Erfindung betrifft ein Verfahren zur Vorhersage und Lokalisierung struktureller Veränderungen im Myokardgewebe (dazu zählen Ischämie-bedingte, entzündungsbedingte, sowie belastungsbedingte Schädigungen der Zellstruktur) mittels Techniken des maschinellen Lernens (Künstliches Neuronales Netzwerk).

### Beschreibung und Einleitung des allgemeinen Gebietes der Erfindung

Der Herzinfarkt ist eine der Haupttodesursachen in den Industrienationen und zählt zur Gruppe der ischämischen Herzerkrankungen. Die Inzidenz beträgt in Österreich/Deutschland etwa 300 Infarkte jährlich pro 100.000 Einwohner (in Japan < 100; Mittelmeer, Schweiz, Frankreich < 200; 300 bis 400 in Skandinavien; 400 bis 500 in England, Ungarn), in Deutschland erleiden jedes Jahr etwa 280.000 Menschen einen Herzinfarkt. Laut Todesursachenstatistik des Statistischen Bundesamtes starben in Deutschland im Jahr 2015 über 49.000 Menschen infolge eines akuten Herzinfarktes. Hierbei ist zu beachten, dass bei bis zu 25 % aller Herzinfarkte nur geringe oder keine Beschwerden auftreten (sogenannte stumme Infarkte). Strukturelle Veränderungen im Myokardgewebe können ein Indikator für ischämische Herzerkrankungen sein, die wiederum eine der Hauptursachen für Herzinsuffizienz darstellen. Üblicherweise erfolgt die Diagnostik solcher Veränderungen, wie z. B. myokardialen Narben, über eine hochauflösende kardiale Bildgebung wie eine MRT (Magnetresonanztomographie)-Untersuchung. Hier bestehen praktische Einschränkungen durch in der Breite nutzbare Werkzeuge zur Diagnose und Risikoabschätzung, da die auf MRT basierenden Techniken aufgrund der Kosten und des erforderlichen Fachwissens weder bettseitig, noch ambulant eingesetzt werden können.

### Stand der Technik

Um geschädigtes Myokardgewebe zu identifizieren, wurde 1989 ein Verfahren auf Basis der zeitlichen Beurteilung der Anreicherung von Gadolinium im Gewebe (sogenannten Late-Gadolinium-Enhancement (LGE)) eingeführt. Diese Technik basiert auf dem Einsatz der Magnetresonanztomographie (MRT) und Gadolinium-basiertem Kontrastmittel. Das Verfahren ist seitdem als Routinesequenz in der kardialen Magnetresonanz-Bildgebung etabliert, um bspw. Narben mit hoher Genauigkeit zu erkennen und zu definieren.

Neben der Bildgebung wird traditionell das Vorhandensein struktureller Veränderungen im Myokardgewebe auf Basis des 12-Kanal-Elektrokardiogramm (EKG) abgeschätzt. Diese Abschätzung basiert auf einer manuellen Analyse. Es existieren derzeit keine etablierten maschinell ausgewerteten Screening-Verfahren hinreichender Genauigkeit.

Im Stand der Technik sind bereits auf maschinellem Lernen basierende Screening-Verfahren für die Erkennung kardialer Rhythmusstörung wie Vorhofflimmern (AF) bekannt. In der Studie von Hannun et al. (Hannun et al. (2019), "Cardiologist-level arrhythmia detection and classification in ambulatory electrocardiograms using a deep neural network", Nature Medicine, 25, 65-69. DOI: 10.1038/s41591-018-0268-3) wird beispielsweise ein Verfahren beschrieben, welches ein Ein-Kanal-EKG im Hinblick auf zwölf daran erkennbare Herzrhythmusstörungen (darunter Vorhofflimmern und -flattern, AV-Block Typ I und II und totaler Herzblock, Bigeminus, Trigeminus, ventrikuläre und supraventrikuläre Tachykardien und junktionaler Rhythmus, sowie Sinusrhythmus und Messfehler ("Noise")) auswertet. Dabei wird ein Künstliches Neuronales Netz mithilfe einer Vielzahl von Befunden trainiert, um im EKG Signal relevante Muster zu erkennen und auszuwerten. Dieses Verfahren lässt jedoch keine direkten Rückschlüsse auf den Zustand des Herzmuskels oder die Lokalisation der erkannten Störungen zu. Es existieren derzeit keine vergleichbaren Verfahren für die Lokalisierung struktureller Veränderungen im Myokardgewebe.

Ein weiteres Verfahren wird von Attia et al. (Attia et al. (2019), "Screening for cardiac contractile dysfunction using an artificial intelligence-enabled electrocardiogram".

Nature Medicine 25, 70-74. DOI:10.1038/s41591-018-0240-2) beschrieben. Hierbei wird mittels künstlicher Intelligenz aus EKG-Daten eine asymptomatische linksventrikuläre Dysfunktion erkannt. Hier wird die über das Echokardiogramm festgestellte Pumpfunktion des Herzens via EKG vorhergesagt. Es werden jedoch auch hier keine Aussagen über den Zustand einzelner Bereiche des Myokards getroffen. Um die nötige diagnostische Präzision und Robustheit zur Erkennung struktureller Veränderungen im Myokardgewebe zu erreichen, sind derzeit einerseits kostenintensive Methoden notwendig, welche zudem fachärztliche Expertise verlangen. Andererseits existieren weitere Methoden, welche eine niedrigere Präzision erreichen, und/oder zeitaufwändig sind und sich daher im klinischen Alltag nicht etablieren konnten. Hierzu zählt der Selvester QRS-Score (Selvester et al. (1985), "The Selvester QRS Scoring System for Estimating Myocardial Infarct Size: The Development and Application of the System". Archives of Internal Medicine, 145, 1877-1881. DOI: 10.1001/archinte.1985.00360100147024), welcher Infarkt-bedingte Schädigungen des Myokards aufgrund des EKGs quantifizieren und lokalisieren kann. Dies ist mit hohem manuellem Aufwand (präzises Ausmessen der EKG-Kurve notwendig, zahlreiche Berechnungen) verbunden und hat sich im klinischen Alltag als wenig praktikabel erwiesen.

US 2019/0090774 A1 beschreibt ein Verfahren zur Bestimmung des Arrhythmie-Ortes, um den Ursprungsort einer Arrhythmie im Herzen eines Probanden zu identifizieren, indem die Identifikation des Herzsegments, in dem die Arrhythmie ihren Ursprung hat, von neuronalen Netzwerken empfangen wird, denen elektrische Daten zugeführt werden. Es wird erwähnt, den linken Ventrikel gemäß der AHA-standardisierten Myokardsegmentierung in 17 Segmente zu unterteilen.

### Aufgabe

Aufgabe der vorliegenden Erfindung ist es, die Nachteile des Standes der Technik zu vermeiden, sodass EKG-Aufzeichnungen und weitere klinische Parameter unter Nutzung eines Künstlichen Neuronalen Netzwerkes (KNN) zur Lokalisierung struktureller Veränderungen im Myokardgewebe genutzt werden können. Damit wird eine präzise und zuverlässige Diagnose ermöglicht, welche derzeit nur mit bildgebenden Verfahren (bspw. MRT) möglich ist.

### Lösung der Aufgabe

Die Lösung dieser Aufgaben ergibt sich aus den Merkmalen des Hauptanspruchs. Weiterhin sind vorteilhafte Ausgestaltungen und Weiterbildungen der Erfindung den Unteransprüchen entnehmbar.

Zunächst erfolgt eine Definition, wie einige für das erfindungsgemäße Verfahren maßgebliche Begriffe im Rahmen dieser Erfindung zu verstehen sind:

### EKG:

EKG steht für Elektrokardiographie und bezeichnet eine Untersuchungsmethode, bei der die elektrische Aktivität des Herzens auf der Körperoberfläche gemessen wird. Nerven- und Muskelzellen verständigen sich über elektrische und chemische Signale. Regelmäßige elektrische Impulse steuern auch den Herzschlag. Sie werden vom sogenannten Sinusknoten im rechten Vorhof des Herzens ausgelöst und breiten sich wie kleine Stromstöße über den Herzmuskel aus. Dadurch ziehen sich zuerst die Vorhöfe und dann die Herzkammern zusammen. Die Ausbreitung der elektrischen Reize im Herzmuskel ist auch auf der Haut messbar. Ein EKG misst diese elektrischen Potentialunterschiede an verschiedenen Stellen des Körpers und stellt diese als Kurven dar. Diese EKG-Kurven werden Elektrokardiogramm genannt.

Wenn das Herz gleichmäßig schlägt, ergibt sich das typische EKG-Muster: Der erste Ausschlag (p-Welle) zeigt, wie sich der elektrische Impuls (Erregung) über die Herzvorhöfe ausbreitet. Die Vorhöfe ziehen sich zusammen, leiten Blut in die Herzkammern und entspannen sich sofort wieder. Die Erregung erreicht dann die Herzkammern. Im EKG ist das als Q-, R- und S-Zacken sichtbar, dem sogenannten QRS-Komplex der mit der Kontraktion der Herzkammern assoziiert ist. Danach zeigt die T-Welle an, dass sich die Erregung zurückbildet und sich die Herzkammern wieder entspannen.

Die zeitlichen eingeordneten Daten des Erregungsablaufes des Herzen werden im Folgenden vereinfacht als EKG- (Mess-) daten bezeichnet.

### MRT:

MRT steht für Magnetresonanztomographie, abgekürzt MRT oder MR. Dies ist ein bildgebendes Verfahren, das vor allem in der medizinischen Diagnostik zur räumlichen Darstellung von Struktur und Funktion der Gewebe und von Organen im Körper eingesetzt wird. Es basiert physikalisch auf den Prinzipien der Kernspinresonanz (englisch Nuclear Magnetic Resonance, NMR), insbesondere der Feldgradienten-NMR, und wird daher auch als Kernspintomographie bezeichnet (umgangssprachlich gelegentlich zu Kernspin verkürzt). Mit der MRT können Schnittbilder des menschlichen (oder tierischen) Körpers erzeugt werden, die eine Beurteilung der Organe und vieler krankhafter Organveränderungen erlauben. Die dabei erzeugten Daten sowie die daraus gewonnenen Interpretationsdaten werden im Folgenden als MRT-Daten bezeichnet.

### Myokard:

Myokard: Der Begriff Myokard bezeichnet den Herzmuskel. Das Myokard bildet den größten Teil des Herzens. Die Herzmuskulatur wird außen vom Epikard und innen von der Herzinnenhaut (Endokard) gebildet und umgeben. Der Herzmuskel ist ein Hohlmuskel, der einen für seine Kontraktion mit Volumenverringerung des Hohlraumes spezifischen makroskopischen (schlingenförmigen, vernetzten) Aufbau besitzt. Die Muskelzüge der Herzkammern ziehen oberflächlich (subepikardial) zur Herzspitze und schlagen am Herzwirbel (vortex cordis) nach innen und ziehen als tiefe (subendokardiale) Muskelschicht zurück zum Herzskelett.

### Myokardiale Pathologie:

Der Begriff myokardiale Pathologie umfasst verschiedene krankhafte Veränderungen des Myokards, dazu zählen unter anderem: Narben, Perfusionsdefizite, Entzündungen und Hypertrophien. Diese können einen oder mehrere Segmente des Myokards umfassen.

### Lokalisation:

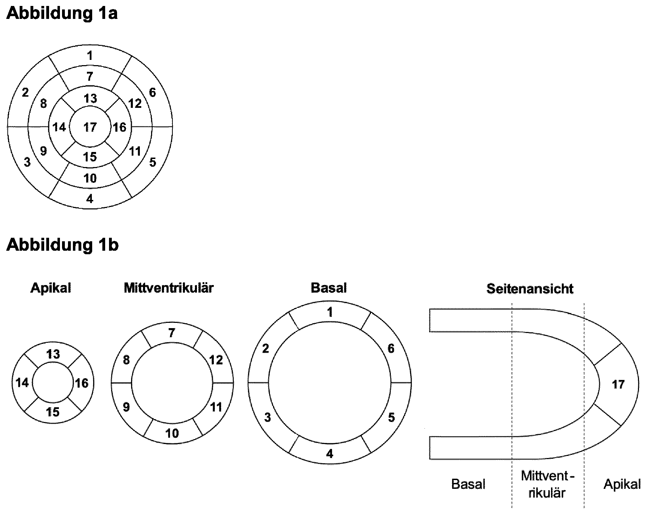

Für diagnostische Zwecke kann das Herz in mehrere Bereiche unterteilt werden. Breite Anwendung findet das 17-Segment-Modell, das von der American Heart Association (AHA) vorgeschlagen wurde. Der Fokus dieses Modells liegt ausschließlich auf dem linken Ventrikel, welcher den signifikantesten Teil des Myokards ausmacht. Der Ventrikel ist in dem Modell zu gleichen Teilen aufgeteilt, senkrecht zur Längsachse des Herzens. Diese basalen, mittkavitären- und apikalen Scheiben sind dann weiter in kleinere Teile aufgeteilt. Jedes Segment wird aufgrund zweier Kriterien benannt: Position auf der Längsachse und Umfangsposition. Diese Segmente können als Bull's-Eye-Diagramm betrachtet werden (siehe Abbildung 1a und Tabelle 1). Mit darauf aufbauender Segmentierung kann man auch die wahrscheinlichen Versorgungsgebiete der jeweiligen Koronararterie definieren, was zu der ebenfalls unter Abbildung 1b angegebene Annäherung führt.

**Tabelle 1:**

| | Basale Segmente (klappennahes Drittel) | | Mittventrikuläre Segmente (mittleres Drittel) | | Apikale Segmente (spitzennahes Drittel) |
|---|---|---|---|---|---|
| 1. | basal anterior | 7. | mittanterior | 13. | apikal anterior |
| 2. | basal anteroseptal | 8. | mittanteroseptal | 14. | apikal septal |
| 3. | basal inferoseptal | 9. | mittinferoseptal | 15. | apikal inferior |
| 4. | basal inferior | 10. | mittinferior | 16. | apikal lateral |
| 5. | basal inferolateral | 11. | mittinferolateral | 17. | apex |
| 6. | basal anterolateral | 12. | mittanterolateral | | |

### Klinische Parameter:

Die Auswertung der EKG-Messwerte kann durch Hinzunahme zusätzlicher klinischer Parameter verbessert werden. Diese beziehen sich insbesondere auf die klassischen kardio-vaskulären Risikofaktoren (siehe Yusuf et al. (2019), "Modifiable risk factors, cardiovascular disease, and mortality in 155722 individuals from 21 high-income, middle-income, and low-income countries (PURE): a prospective cohort study". The Lancet, Sep 03, 2019. DOI: 10.1016/S0140-6736(19)32008-2).

Ein wichtiger Risikofaktor ist das Vorhandensein einer Atherosklerose. Atherosklerose ist ursächlich für die Mehrheit der ischämischen kardiovaskulären Erkrankungen, wie z.B. Myokardinfarkt. Diese progressive Krankheit ist gekennzeichnet durch eine Anhäufung von Lipiden und Faserelementen in den großen Arterien. Der Grund dafür ist unter anderem eine Entzündungsreaktion, die zu einer Veränderung und einer Fehlfunktion des arteriellen Endothels führt. Dies führt zur Bildung von sogenannten Plaques innerhalb der Arterienwände. Diese Plaques reduzieren das arterielle Lumen und bergen das Risiko von Rissen, was zu einem schweren thrombotischen Verschluss der betroffenen Arterie führen kann. Dieser führt schlussendlich zu einem Infarkt des abhängigen Gewebes. Atherosklerose ist eine komplexe Erkrankung, bei der sich mehrere Prozesse gegenseitig beeinflussen.

Weitere wichtige klinische Parameter sind im Folgenden beschrieben.

Dyslipidämie, auch Fettstoffwechselstörung genannt, ist gekennzeichnet durch eine abnorme Zusammensetzung der Lipide (vereinfacht in diesem Zusammenhang oftmals als Fette bezeichnet) im Blut. Ein wichtiges Lipid ist Cholesterin. Lipide erfüllen lebenswichtige Aufgaben, wie Energiespeicherung, Bildung von Zellmembranen, etc. und dienen als Botenstoffe. Ein hoher Anteil an Lipiden kann die Bildung atherosklerotischer Plaques begünstigen. Der Lipidspiegel im Blut wird durch die Absorption im peripheren Gewebe reguliert. Die Leber ist verantwortlich für die Regulierung der Lipidwerte durch Sekretion oder Absorption. Wenn dieser Mechanismus beeinträchtigt ober überbeansprucht ist, führt das zu einem erhöhten Lipidspiegel. Dies kann dazu führen, dass sich mehr und mehr atherosklerotische Plaques bilden und sich das Risiko eines Risses weiter erhöht.

Übergewichtige Menschen haben übermäßiges Fettgewebe, was zu einer Zunahme von entzündungsfördernden Zytokinen führt. Diese Zytokine fördern das Voranschreiten einer Atherosklerose und Insulinresistenz. Übergewicht hat darüber hinaus negative Auswirkungen auf die arterielle Hypertonie und den Diabetes mellitus, was es zu einem besonders wichtigen klinischen Parameter macht.

Im Allgemeinen werden arterielle Hypertonie, Diabetes mellitus und Dyslipidämie oft zusammen mit Übergewicht mit dem so genannten Metabolischen Syndrom in Verbindung gebracht, wobei sich diese Faktoren gegenseitig verstärken.

Ein weiterer wichtiger klinischer Parameter ist das Vorhandensein von arterieller Hypertonie (Bluthochdruck). Er erhöht die Belastung des Endothels. Arterielle Hypertonie belastet das Myokard zusätzlich. So leiden Patienten oftmals sowohl an Atherosklerose als auch an erhöhtem Blutdruck. Das Myokard hat dadurch einerseits mit weniger Sauerstoff und andererseits mit höherer physischer Belastung zu kämpfen. Das führt zu einer Diskrepanz zwischen Sauerstoffangebot und -bedarf, was zu Ischämie und Nekrose führen kann.

Rauchen ist ein weiterer wichtiger Parameter. Rauchen schadet dem Endothel und erhöht die Aggregation der Blutplättchen (Thrombozyten)und die Fibrinogenkonzentration im Blut (bezeichnet als Hyperkoagulabilität). Weiterhin wird die Menge der Highdensity Lipoproteine, die für den Transport von übermäßigem Cholesterin zurück zur Leber sorgen, durch das Rauchen reduziert. Dies begünstigt die Weiterentwicklung von Plaques und erhöht das Risiko einer Ruptur und eines Verschlusses betroffener Arterien.

Ein weiterer wichtiger Parameter ist das Vorhandensein von Diabetes mellitus. Dies ist eine komplexe Stoffwechselerkrankung, die mit einem erhöhten Risiko beispielsweise für das Erleiden eines Myokardinfarktes verbunden ist. Es wird unterschieden zwischen Typ 1 und Typ 2-Diabetes. Typ 1 basiert auf einer chronischen Autoimmunerkrankung, die ein ausreichendes Funktionieren des Insulins verhindert. Typ 2 wird auch als erworbener Diabetes bezeichnet, da dieser abhängig vom Lebensstil (Ernährung, Aktivität) ist und sich damit beeinflussen lässt. Schäden entstehen dabei durch direkte und indirekte Einflüsse. Durch reduzierte Empfindlichkeit gegenüber Insulin oder geringer Sekretion von Insulin ist der Blutzuckerspiegel dauerhaft erhöht. Dadurch werden die Zellen einem erhöhten osmotischen Stress ausgesetzt, woraufhin ihre Struktur beschädigt werden kann, was bis zu einer Nekrose des betroffenen Gewebes führen kann. Dies kann ähnliche Auswirkungen haben wie ein Infarkt durch Atherosklerose. Diabetes mellitus hat weiterhin auch einen negativen Einfluss auf das Endothel und begünstigt die Atherogenese, insbesondere dann, wenn sie gemeinsam mit erhöhten Lipidwerten auftritt.

Die genetische Disposition hat einen nachgewiesenen Einfluss auf Herz-Kreislauf-Erkrankungen und ist damit auch ein wichtiger klinischer Parameter. So haben mehrere Studien gezeigt, dass ein erhöhtes Risiko für das Auftreten struktureller Veränderungen im Myokardgewebe für Menschen besteht, deren Angehörige erster Ordnung einen Myokardinfarkt vor dem 55. Lebensjahr erlitten haben. Die genomweite Assoziationsanalyse wird verwendet, um das Auftreten genetischer Defekte oder Mutationen, die für ein erhöhtes Risiko für die Entwicklung bestimmter Krankheiten verantwortlich sein könnten, zu identifizieren. Mehrere Gene wurden identifiziert, die mit erhöhter Anfälligkeit für Diabetes mellitus und beschleunigter Atherogenese in Zusammenhang gebracht werden. Es konnte gezeigt werden, dass ein Hang zu Hauptstammstenosen der Koronararterien, deren Verkalkung, sowie deren Läsionen (Aneurysma/Ektasie) erblich sind, was direkten Einfluss auf das Risiko für koronare Herzkrankheiten und Myokardinfarkt hat. Es wurde ferner festgestellt, dass die genetischen Faktoren unabhängig von anderen Risikofaktoren sind.

Ein weiterer wichtiger Parameter ist das Geschlecht des Patienten, da geschlechtsspezifische Unterschiede auch in der Prävalenz von kardiovaskulären Erkrankungen vorhanden sind. Basierend auf verschiedenen Studien konnte beobachtet werden, dass der erste Myokardinfarkt bei Frauen im Durchschnitt neun Jahre später auftritt als bei Männern. Prämenopausale Frauen zeigen eine reduzierte Prävalenz kardiovaskulärer Ereignisse als Männer im gleichen Alter. Dieser Unterschied verringert sich mit der Menopause. Dies kann dadurch erklärt werden, dass Östrogene vasoaktive Hormone sind, die direkte Auswirkungen auf die Arterienwand haben. Sie schützn das Endothel durch Erhöhung der Produktion von Stickstoffmonoxid (NO), was den oxidativen Stress reduziert. Sie regulieren weiterhin indirekt den Lipidspiegel im Blut.

Das Alter wirkt auf verschiedene Art und Weise auf die Eigenschaften von Endothel und Blut ein. Die endotheliale Funktion ist mit zunehmendem Alter beeinträchtigt. Das Altern verringert die Produktion von NO, was zu einem erhöhten Blutdruck führt und die Endothelfunktion beeinträchtigt. Aufgrund der altersbedingten Stoffwechselveränderungen werden die Gerinnungsfähigkeit des Blutes, sowie auch das Risiko für Thrombosen, erhöht.

Diese Aufzählung nennt nur beispielhaft wichtige klinischen Parameter zur Identifizierung kardialer Pathologien und ist nicht abschließend zu verstehen.

### Künstliche Neuronale Netze:

Künstliche Neuronale Netze, auch Künstliche Netzwerke, kurz: **KNN** (englisch artificial neural network, ANN), sind Netze aus künstlichen Neuronen. Künstliche Neuronale Netze basieren auf der Vernetzung vieler einzelner, meist in Schichten angeordneter, künstlicher Neuronen. Die Intensität der Verbindungen zwischen den Neuronen wird durch sogenannte Gewichtungen/Gewichte gesteuert. Die Topologie sowie die steuernden Parameter eines Netzes werden in Abhängigkeit der Problemstellung gewählt.

In einer Trainingsphase werden KNN anhand von Eingangsdaten (Eingangssignal), für die erwartete Ergebnisse (Soll-Signal) vorliegen, durch Vergleich dieser mit den errechneten Ergebnissen (Ergebnis-Signal) angelernt.

Die Differenz von Ergebnis- und Soll-Signal wird als Loss bezeichnet. Der Loss zeigt an, wie gut das erwartete und das tatsächliche Ergebnis übereinstimmen. Um die Qualität der Verarbeitung zu verbessern, muss dieser Loss für zukünftige Durchläufe berücksichtigt werden. Diese Anpassung der Verarbeitung bezeichnet man als Backpropagation bzw. Gewichts-Anpassung. Hierbei werden die Gewichte zwischen den Neuronen derart justiert, dass sie zukünftig dem gewünschten Ergebnis näherkommen.

Basierend darauf kann die Lernfähigkeit von KNNs als Fähigkeit zum Vergleich eines bestimmten Ergebnisses und eines erwarteten Ergebnisses, gefolgt von einer Anpassung entsprechend der Differenz zwischen diesen Werten, definiert werden.

KNNs sind dadurch in der Lage, mithilfe eines iterativen Trainingsprozesses komplizierte nichtlineare Funktionen zu erlernen.

Ein weit verbreitetes Prinzip für das Training von KNN stellt das überwachte Lernen dar. Hierbei wird das Neuronale Netz mit einer Menge von Tupeln aus zusammengehörenden Eingangs- (x) und Ausgangssignalen (y) mithilfe des zuvor beschriebenen Prinzips trainiert und iterativ angepasst. Die Menge von Tupeln (x, y), die für das Training genutzt werden, bezeichnet man als Trainingsdaten (D_{training}). Die Funktion des Netztes auf ihm unbekannten Daten wird mithilfe von Validierungsdaten (D_{validation}) ermittelt. Diese Menge von Tupeln (x, y) enthält Daten, mit denen das Netzt nicht trainiert wurde. Es gilt D_{training} ∩ D_{validation} = Ø.

### Erfindungsgemäßes Verfahren:

Die in einem vorangegangenen Schritt (der nicht Teil des erfindungsgemäßen Verfahrens ist) erhobenen EKG-Trainingsmessdaten TD1 und klinischen Parameter D2 werden als Eingangssignale des erfindungsgemäßen Verfahrens verwendet.

Die klinischen Parameter D2 sind optional und werden - nach eventueller Imputation - in die Verarbeitung mit einbezogen. Imputation bedeutet, dass fehlende Daten für die Parameter D2 (unvollständige Datensätze) in statistischen Erhebungen - die sogenannten Antwortausfälle - in der Datenmatrix vervollständigt werden. Die Schweigeverzerrung, die durch die Antwortausfälle entsteht, wird dadurch verringert. Es gibt dabei verschiedene Verfahren, mit denen fehlende Werte vervollständigt werden allgemein bekannt. Dabei unterscheidet man grob zwischen der singulären und der multiplen Imputation.

Für das Training des neuronalen Netzes werden bevorzugt wenigstens ein klinischer Parameter D2, ein EKG-Ausschnitt TD1, sowie die auf Basis eines MRT gestellte Diagnoseinformation D3 genutzt. Die klinischen Parameter wie auch der EKG-Ausschnitt stellen dabei die Eingangssignale (TD1, D2) dar, während die Diagnose D3 die Zielwerte für die Ausgangs-Signale des Netzes bereitstellt. Der Diagnose-Datensatz D3 beinhaltet dabei Indikatoren für unterschiedliche Gewebeveränderungen inklusive deren Lokalisierung. Diese Menge von Tupeln aus (x, y) bilden die Trainingsdaten (D_{training}). Hierbei entspricht ein x einem Tupel aus den Daten TD1 und vorzugsweise D2 (EKG-Ausschnitt, klinische Parameter) und ein y einem Vektor mit der Diagnoseinformation D3. Beim EKG-Ausschnitt handelt es sich um eine Matrix aus #LeadsEKG und #SequenzlängeEKG, während die klinischen Parameter D2 in Form eines Vektors mit den Parametern als Einträge bereitgestellt werden.

Aus den Eingangssignalen x wird über das KNN ein Ergebnissignal y' ermittelt, was wie zuvor beschrieben mit dem wahren y abgeglichen und zur Berechnung des Loss genutzt wird.

### Zum Training des neuronalen Netzwerks:

Aus dem Vergleich der aus TD1 und vorzugsweise D2 berechneten Ist-Werte mit den Soll-Werten D3 der Trainingsdaten, werden die im Künstlichen Neuronalen Netzwerk enthaltenen Gewichte angepasst. Dieser iterative Prozess endet mit einem autark anwendbaren neuronalen Netz N.

Hierdurch wird das Netz N zur Mustererkennung befähigt. Bei der Nutzung von N zur Vorhersage erfolgt daher kein Zugriff auf konkrete Datensätze der Trainingsdaten, sondern ausschließlich auf die erlernten Gewichte. Die Vorhersage erfolgt damit nur auf Basis dieser durch das Training erlernten Gewichte.

### Trainingsphase

Vor der eigentlichen Durchführung des erfindungsgemäßen Verfahrens muss das KNN eine Trainingsphase als einen Schritt 0 durchlaufen.

Dieser Schritt 0 umfasst folgende Teilschritte:
**0a.** Empfangen der EKG-Trainingsmessdaten TD1.
**0b.** Vorverarbeitung der EKG-Trainingsmessdaten TD1 zu vorverarbeiteten EKG-Trainingsmessdaten TD1* und Übergabe dieser vorverarbeiteten EKG-Trainingsmessdaten TD1* an das Künstliche Neuronale Netz N.

### Vorverarbeitung in der Trainingsphase:

Um für das Training des Netztes qualitativ hochwertige Daten zu nutzen, müssen die verwendeten Daten wie bspw. EKG-Daten TD1 bzw. klinischen Parameter D2 zu vorverarbeiten EKG-Daten TD1* bzw. vorverarbeiteten klinischen Parameter D2* aufbereitet werden. Dies wird auch als Vorverarbeitung bezeichnet. Dies wird schematisch in Abbildung 2 gezeigt.

Dazu muss eine sogenannte Daten-Pipeline eingerichtet werden. Diese definiert die einzelnen Teilschritte der Vorverarbeitung. Diese Daten-Pipeline dient dazu, die heterogen gespeicherten Daten in bereinigte Datensätze zu überführen, die problemlos im weiteren Verfahren zu verwenden sind.

Die Vorverarbeitung berücksichtigt geeignete Maßnahmen zur Anreicherung (Data Augmentation) der Daten.

Die Vorverarbeitung in der Trainingsphase umfasst folgende Schritte:

### a. Extraktion

Die EKG-Daten der einzelnen Patienten werden aus den Rohdaten extrahiert. Die EKG-Daten liegen dabei üblicherweise im XML-Dateiformat vor.

Die optionalen klinischen Parameter der einzelnen Patienten sowie die auf Basis des MRT getroffenen Diagnosen werden aus den Patientenrohdaten-Dateien extrahiert. Diese liegen üblicherweise im CSV-Dateiformat vor. Anschließend werden die EKG-Daten, die klinischen Parameter und die Diagnose-Informationen je eines Patienten zu einem Tupel (x, y) verknüpft.

### b. Auswahl

In diesem Schritt werden nicht verwendbare Datensätze von der weiteren Verarbeitung ausgeschlossen (z. B. unvollständige klinische Parameter, die nicht imputiert werden können). Es sollen nur vollständige Datensätze für das Training genutzt werden, um das Erlernen von Korrelationen zu optimieren.

Es können alle EKG-Ableitungen verwendet werden, es muss keine Auswahl von spezifischen Ableitungen vorliegen.

### c. Bereinigung

Die EKG-Aufzeichnungen werden beschnitten, um technische Artefakte z.B. am Beginn und/oder am Ende zu beseitigen.

### d. Augmentierung

Um die für das Training verwendbare Datenmenge zu erhöhen und dadurch die Performance des Netzes zu steigern, werden eindeutige Teilausschnitte aus jeder EKG-Aufnahme extrahiert. Hierbei wird ein Fenster fixer Größe Schritt für Schritt über die Aufnahme bewegt, was jeweils eine neue Aufnahme generiert. Dies ermöglicht das Erhöhen der Datenmenge um bspw. den Faktor 100.

### e. Formatierung

Um hochfrequentes Rauschen und die Größe der EKG-Aufnahmen zu reduzieren, wird für jeden Datensatz ein Down-Sampling durchgeführt. Dies reduziert durch ein Average-Pooling-basiertes Verfahren Details der Aufnahme, erhält jedoch deren signifikante Muster. Hierdurch lässt sich die Datengröße um bspw. den Faktor 4 reduzieren.

### f. Speichern

Die Daten werden in gleichgroße Gruppen aufgeteilt. Abhängig vom gewünschten Verhältnis zwischen Trainingsdaten und Validierungsdaten (bevorzugt ist hierbei ein Verhältnis von 80% zu 20%) wird eine bestimmte Anzahl von Gruppen (z.B. 6) für das Training verwendet, der Rest für die Validierung. Diese Konstellation aus Trainings- und Validierungssplit wird je als eine Datensatz-Variante gespeichert. Dieser Vorgang wird so lange wiederholt, bis entweder genügend Varianten angelegt sind oder alle Kombinationen von Gruppen im Training/Validierung aufgetreten sind. Dies ermöglicht das Training identisch aufgebauter Modelle mit unterschiedlichen Daten. In Kombination liefern diese Modelle (z.B. 6) eine höhere Performance auf unbekannte Daten als ein einzelnes Modell aus dieser Menge. Man bezeichnet dieses Verfahren als Ensembling.
**0c**. Ermittlung der Wahrscheinlichkeit, welchen Prädiktionswert wenigstens eine kardiale Pathologie für wenigstens ein Segment des Myokards aufweist durch das Künstliche Neuronale Netz N.
**0d**. Eingabe der MRT-Diagnose-Informationen D3.

Die MRT-Diagnose-Informationen D3 dienen als Referenzdaten zum Training des KNN. Alternativ können auch Daten aus anderen bildgebenden Verfahren als Referenzdaten verwendet werden.
**0e**. Iterativer Soll-Ist-Vergleich der ermittelten Wahrscheinlichkeiten mit den MRT-Diagnose-Informationen D3 mit anschließendem Anpassen der Gewichte G im Künstlichen Neuronalen Netz N.
**0f**. Fertigstellen des trainierten Künstlichen Neuronalen Netzes N den angepassten Gewichten G.

In einer Weiterbildung des Trainingsverfahrens erfolgt optional vor dem Schritt 0b noch ein zusätzlicher Schritt 0a* in welchem wenigstens ein klinischer Parameter D2 empfangen werden. In diesem Fall erfolgt anschließend ein zusätzlicher Schritt 0b*, welcher zwischen Schritt 0a* und Schritt 0c stattfindet. Schritt 0b* umfasst eine Vorverarbeitung des wenigstens einen klinischen Parameter D2 zu einem vorverarbeiten klinischen Parameter D2* und eine Übergabe dieses wenigstens einem vorverarbeiten klinischen Parameter D2* an das Künstliche Neuronale Netz N. Durch das Hinzuziehen der klinischen Parametern D2 kann die Zuverlässigkeit der Trainingsphase erhöht werden.

Nach der Trainingsphase wird das erfindungsgemäße Verfahren durchgeführt.

Das erfindungsgemäße Verfahren umfasst die folgenden Schritte:

### I. Bereitstellen der EKG-Messdaten D1

In einem ersten Schritt werden EKG Messdaten bereitgestellt. EKG-Messdaten sind dabei die Daten, welche nach Abschluss der EKG-Messung am Patienten vorliegen. Das Bereitstellen beginnt erst nach Abschluss der Untersuchung mit Datenerhebung am menschlichen oder tierischen Körper, d.h. erst nachdem die Messung am Patienten abschlossen ist.

Für die Durchführung des Verfahrens erfolgt keine Messung am Patienten selbst. Das Verfahren kann sogar durchgeführt werden, wenn der Patient bereits verstorben ist.

### II. Vorverarbeiten der EKG-Messdaten D1 und Übergabe dieser Daten an ein Künstliches Neuronales Netz N

### Vorverarbeitung im erfindungsgemäßen Verfahren:

Auch für das erfindungsgemäße Verfahren müssen die verwendeten Daten wie bspw. EKG-Daten D1 und sofern vorhanden klinischen Parameter D2 zu vorverarbeiteten EKG-Daten D1* und vorverarbeiteten klinischen Parameter D2* aufbereitet werden, sodass sie an ein Künstliches Neuronales Netz übergeben werden können. Dies wird auch als Vorverarbeitung bezeichnet.

Dazu muss eine sogenannte Daten-Pipeline eingerichtet werden. Diese definiert die einzelnen Teilschritte der Vorverarbeitung. Diese Daten-Pipeline dient dazu, die heterogen gespeicherten Daten in bereinigte Datensätze zu überführen, die problemlos im weiteren Verfahren zu verwenden sind.

Die Vorverarbeitung berücksichtigt geeignete Maßnahmen zur Anreicherung (Data Augmentation) der Daten.

Die Vorverarbeitung umfasst folgende Schritte:

### a. Extraktion der EKG-Daten des einzelnen Patienten

Die EKG-Daten des einzelnen Patienten werden aus den Rohdaten extrahiert. Die EKG-Daten liegen dabei üblicherweise im XML-Dateiformat vor.

Die optionalen klinischen Parameter der einzelnen Patienten werden aus den Patientenrohdaten-Dateien extrahiert. Diese liegen üblicherweise im CSV-Dateiformat vor. Anschließend werden die EKG-Daten, die klinischen Parameter und die Diagnose-Informationen je eines Patienten zu einem Tupel (x, y) verknüpft.

### b. Auswahl

In diesem Schritt werden nicht verwendbare Datensätze von der weiteren Verarbeitung ausgeschlossen (z. B unvollständige klinische Parameter, die nicht imputiert werden können). Es sollen nur vollständige Datensätze für das Verfahren genutzt werden, um die Verwendung zu optimieren.

Es können alle EKG-Ableitungen verwendet werden, es muss keine Auswahl von spezifischen Ableitungen vorliegen.

### c. Bereinigung

Die EKG-Aufzeichnungen werden beschnitten, um technische Artefakte z.B. am Beginn und/oder am Ende zu beseitigen.

### d. Augmentierung

Um die verwendbare Datenmenge zu erhöhen und dadurch die Performance des Netzes zu steigern, werden eindeutige Teilausschnitte aus jeder EKG-Aufnahme extrahiert. Hierbei wird ein Fenster fixer Größe Schritt für Schritt über die Aufnahme bewegt, was jeweils eine neue Aufnahme generiert. Dies ermöglicht das Erhöhen der Datenmenge um bspw. den Faktor 100.

### e. Formatierung

Um hochfrequentes Rauschen und die Größe der EKG-Aufnahmen zu reduzieren, wird für jede Aufnahme ein Down-Sampling durchgeführt. Dies reduziert durch ein Average-Pooling-basiertes Verfahren Details der Aufnahme, erhält jedoch deren signifikante Muster. Hierdurch lässt sich die Datengröße um bspw. den Faktor 4 reduzieren.

### f. Speichern der vorverarbeiteten EKG-Daten D1*

Die vorverarbeiteten EKG-Daten D1* werden abschließend abgespeichert, sodass sie für die Anwendung eines Künstlichen Neuronalen Netzes zur Verfügung stehen.

### III. Anwendung des trainierten Künstlichen Neuronalen Netzes N auf die vorverarbeiteten EKG-Daten D1* zur Ermittlung der Ergebnisdaten E, welche wenigstens einen Prädiktionswert für wenigstens eine kardiale Pathologie für wenigstens ein Segment des Myokards umfassen

Dabei werden die vorverarbeiteten EKG-Daten D1* über den gesamten Verlauf einer EKG-Messung genutzt und nicht nur einzelne typischen Ausschläge, wie dies typischerweise von Ärzten bei einer manuellen Auswertung der Fall ist. Damit trägt die Gesamtheit der EKG-Daten zur Ermittlung der Ergebnisdaten bei. Die Abtastfrequenz für EKG-Geräte beträgt mindestens 50 Hz. Vorzugsweise weisen sie jedoch eine Abtastfrequenz von mindestens 500 Hz auf. Da alle Messpunkte der vorverarbeiteten EKG-Daten D1* verwendet werden beträgt die Abtastfrequenz der vorverarbeiteten EKG-Daten D1* ebenfalls mindestens 50 Hz. Bevorzugt beträgt die Abtastfrequenz vorverarbeiteten EKG-Daten D1* mindestens 500 Hz.

Dies ermöglicht eine besonders präzise und zuverlässige Ermittlung von Ergebnisdaten.

### IV. Ausgabe der Ergebnisdaten E

Dabei wird in Schritt III das trainierte Künstliche Neuronale Netz N auf die vorarbeiteten EKG-Daten D1* mit den in einer Trainingsphase angepassten Gewichten G angewendet. Hierbei werden die vorverarbeiteten Daten D1* und gegebenenfalls wenigstens ein vorverarbeiteter klinischer Parameter D2* als Eingabe an das Netz N übergeben, welches dann durch Anwendung der trainierten Netzstruktur eine Vorhersage p (0<=p<=1) - also einen eindimensionalen Wahrscheinlichkeitswert - für das Vorliegen und optional für die Lokalisation einer kardialen Pathologie berechnet und als Ergebnisdaten E ausgibt.

Der Ergebnisdaten E umfassen wenigstens einen Prädiktionswert für wenigstens eine kardiale Pathologie für wenigstens ein Segment des Myokards, sodass aus diesen Ergebnisdaten E eine Vorhersage und eine Lokalisierung struktureller Veränderungen im Myokardgewebe erfolgen kann. Dieser Prädikationswert für den Ort einer Pathologie ist ein für die Diagnose relevantes Zwischenergebnis, aber muss für eine Diagnose noch von ärztlichen Fachpersonal im medizinischen Kontext interpretiert werden. Ein Prädikationswert an sich stellt damit noch keine Diagnose dar.

Die MRT-Diagnose-Informationen D3 werden nur während der Trainingsphase aber nicht während des erfindungsgemäßen Verfahrens verwendet.

Vorzugsweise werden Prädiktionswerte für alle 17 Segmente des Myokards bestimmt.

Bei der Auswertung der Ergebnisdaten ist entscheidend zu wissen, wie groß die Wahrscheinlichkeit ist, dass ein positives Testergebnis tatsächlich eine kardiale Pathologie bedeutet. Dieser Vorhersagewert des positiven Tests wird auch als (positiver) Prädiktionswert bezeichnet. Die Ergebnisdaten E werden vorzugsweise in Form dieser Prädiktionswerte angegeben. Die Interpretation der Prädikationswerte zur Erstellung einer konkreten Diagnose ist ärztlichem Fachpersonal vorbehalten und nicht Teil des Verfahrens.

Die Schwellwerte können dabei für jede Pathologie und für jedes Herzsegment separat und unabhängig voneinander ausgewählt werden. Die Auswahl der Schwellwerte erfolgt nach der Trainingsphase und vor dem Schritt III des erfindungsgemäßen Verfahrens und hängt von der Art der Pathologie und deren Auswirkungen auf das entsprechende Herzsegment ab. Diese Schwellenwerte werden von ärztlichem Fachpersonal auf Grund ihrer Ausbildung und Erfahrung ermittelt und sind nicht Teil des erfindungsgemäßen Verfahrens. Ein typischer Schwellwert für das Vorliegen einer Pathologie in einem bestimmten Herzsegment kann beispielweise 0,5 sein, das heißt Werte von 0,5 oder größer werden als Pathologien identifiziert.

In einer Weiterbildung des erfindungsgemäßen Verfahrens erfolgt optional vor dem Schritt II noch ein zusätzlicher Schritt la, in welchem wenigstens ein klinische Parameter D2 empfangen wird. In diesem Fall erfolgt in einem anschließenden zusätzlichen Schritt IIa, welcher zwischen Schritt la und Schritt III stattfindet, eine Vorverarbeitung des wenigstens einen klinischen Parameter D2 zu einem vorverarbeiten klinischen Parameter D2*und eine Übergabe dieses wenigstens einem vorverarbeiten klinischen Parameter D2* an das trainierte Künstliche Neuronale Netz N. Dies erfolgt jedoch optimaler Weise dann, wenn der wenigstens eine klinischen Parameter D2 bereits in einer Trainingsphase verwendet werden.

Durch das Hinzuziehen klinischer Parameter D2 kann die Zuverlässigkeit des Verfahrens erhöht werden.

Im Rahmen des erfindungsgemäßen Verfahrens wird auf Basis einer breit verfügbaren Diagnosemethode (EKG) das Ergebnis einer nur eingeschränkt verfügbaren Diagnosemethode (z. B. MRT) vorhergesagt und damit werden die diagnostischen Möglichkeiten des EKG erweitert.

### Vorrichtung:

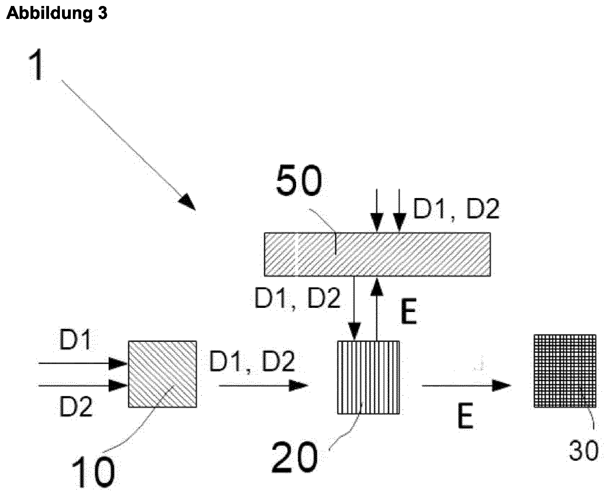

Die erfindungsgemäße Vorrichtung 1 zur Vorhersage spezifischer kardialer Pathologien auf Basis von EKG-Aufzeichnungen und weiterer klinischer Parameter umfasst wenigstens die folgenden Elemente (siehe Abbildung 3):

### Eine Eingabeeinheit 10:

Die Eingabeeinheit 10 ist so ausgebildet, dass über diese EKG-Messdaten D1 und die Daten der klinischen Parameter D2 einzugeben sind und an die Auswerteeinheit 20 zur weiteren Verarbeitung zu übertragen sind. Bei der Eingabeeinheit 10 kann es sich um eine Tastatur oder um eine grafische Benutzeroberfläche (engl. GUI von graphical user interface) handeln. Die Datenübermittlung kann dabei über eine feste Datenleitung (z.B. über eine interne Datenleitung, Local-Area-Network (LAN) etc.), oder drahtlos (z.B. über Wireless Local-Area-Network (WLAN), Bluetooth etc.) erfolgen.

### Eine Datenschnittstelle 50:

Die Datenschnittstelle 50 ist so ausgebildet, dass sie die EKG-Messdaten D1 und die Daten der klinischen Parameter D2 aus einem externen Datenverarbeitungssystem empfangen und die Ergebnisdaten an ein externes Datenverarbeitungssystem weiterleiten kann. Die Datenübermittlung kann dabei über eine feste Datenleitung (z.B. über ein LAN etc.), oder drahtlos (z.B. über WLAN, Bluetooth etc.) erfolgen.

Weiterhin ist die Datenschnittstelle 50 so ausgebildet, dass sie EKG-Messdaten D1 und die Daten der klinischen Parameter D2 an die Auswerteinheit 20 übermitteln kann. Die Datenübermittlung kann dabei über eine feste Datenleitung (z.B. über ein LAN etc.), oder drahtlos (z.B. über WLAN, Bluetooth etc.) erfolgen. Ebenso kann die Übermittlung der Ergebnisdaten E an externe Datenverarbeitungseinheiten über die Datenschnittstelle 50 erfolgen.

Die Datenschnittstelle 50 ist üblicherweise auf den Austausch mit verschiedenen externen Datenverarbeitungssystemen ausgelegt, d.h. gängige Schnittstellen zum Datenaustausch sind bspw. USB, Cardreader, LAN, WLAN, Bluetooth.

Die Datenschnittstelle 50 ist dabei so ausgebildet, dass ein Anwender mit einem beliebigen webfähigen Gerät (bspw. Tablet, PC, Smartphone) im lokalen Netzwerk über die Datenschnittstelle 50 auf die Vorrichtung 1 zugreifen kann.

### Eine Auswertungseinheit 20:

Die Auswertungseinheit 20 ist so ausgebildet, dass sie die EKG-Messdaten D1 und die Daten der klinischen Parameter D2 von der Eingabeeinheit 10 oder einer Datenschnittstelle 50 empfangen kann. Die Datenübermittlung kann dabei über eine feste Datenleitung (z.B. über eine interne Datenleitung, LAN etc.), oder drahtlos (z.B. über WLAN, Bluetooth etc.).

Die Auswertungseinheit 20 ist weiterhin so ausgebildet, dass sie aus den EKG-Messdaten D1 und den klinischen Parametern D2 und aus den mittels Trainingsdaten enthaltenen erlernten Gewichten die Wahrscheinlichkeit und die räumliche Lokalisierung einer kardialen Pathologie des Myokards durch das künstliche Neuronale Netz berechnen kann. Das Ergebnis dieser Berechnung sind dabei die Ergebnisdaten E. Als Auswertungseinheit 20 dient dabei ein Gerät zur elektronischen Datenverarbeitung, z.B. ein in die Vorrichtung 1 integrierter programmierbarer Mikroprozessor.

Die Auswertungseinheit 20 ist weiterhin so ausgebildet, dass sie die Ergebnisdaten E an eine Ausgabeeinheit 30 übermitteln kann. Die Datenübermittlung kann dabei über eine feste Datenleitung (z.B. über eine interne Datenleitung, LAN etc.), oder drahtlos (z.B. über WLAN, Bluetooth etc.) erfolgen.

Die Auswertungseinheit 20 verwendet ein Künstliches Neuronales Netzwerk N zur Mustererkennung. Die Vorrichtung 1 berechnet Prädiktionswerte (eine Liste von Wahrscheinlichkeitswerten für das Vorliegen kardialer Pathologien in den verschiedenen Segmenten des Myokards) als Ergebnisdaten E. Diese Daten sagen aus, wie wahrscheinlich das Vorhandensein resp. Nichtvorhandensein einer spezifischen kardialen Pathologie in einem bestimmten Bereich des Herzens ist. Diese Ergebnisdaten E werden über die Ausgabeeinheit 30 in Form visueller und/oder akustischer Ausgaben ausgeben. Alternativ werden sie an die Datenschnittstelle 50 weitergeleitet.

### Eine Ausgabeeinheit 30:

Die Ausgabeeinheit 30 ist so ausgebildet, dass sie die Ergebnisdaten E der Auswertungseinheit 20 empfangen und dann ausgeben kann. Die Ausgabe kann optisch, akustisch, haptisch und/oder in Form eines Ausdruckes erfolgen.

Die Eingabeeinheit 10 bzw. die Ausgabeeinheit 30 des Gerätes können dabei bspw. Tasten, LEDs, (Touch) Display und Sprachein-/ausgabe umfassen.

In einer ersten alternativen Ausführungsform ist die Vorrichtung 1 so aufgebaut, dass eine komplett gekapselte Verarbeitung der Daten unter Ausschluss jeglicher Netzzugriffe (LAN, WLAN) erfolgen kann. Dazu sind sämtliche Netzwerkkomponenten für jeden Datenaustausch der nicht zwischen Eingabeeinheit 10, Auswertungseinheit 20 und Ausgabeeinheit 30 erfolgt gesperrt.

Mit Krankheitserregern kontaminierte Medizinprodukte (z.B. Messgeräte) können die Quelle von Infektionen beim Menschen sein. Die Anwendung solcher Medizinprodukte setzt daher eine vorhergehende Aufbereitung voraus, an die definierte Anforderungen zu stellen sind.

In einer zweiten alternativen Ausführungsform umfasst die Vorrichtung 1 deshalb ein Gehäuse, um die Auswertungseinheit 20, welches abwaschbar ausgebildet ist. Hierbei sind zusätzlich noch die Eingabeeinheit 10 und die Ausgabeeinheit 30 abwaschbar ausgebildet. Dazu weisen das Gehäuse 10, die Eingabeeinheit 10 und die Ausgabeeinheit 30 vorzugsweise eine Beschichtung aus Parylen oder aus wasserdichtem Silikon auf. Das ermöglicht eine besonders hygienische Verwendung.

(Ab-)Waschbar heißt hierbei: Die Oberfläche ist komplett geschlossen und lässt sich gemäß den Richtlinien des Verbunds für Angewandte Hygiene (VAH) oder der *Deutsche Gesellschaft für Hygiene und Mikrobiologie (DGHM)* mit allen gängigen Desinfektionsmitteln aufbereiten.

Vorzugsweise sind dabei das Gehäuse die Eingabeeinheit 10 und die Ausgabeeinheit 30 sterilisierbar nach DIN EN 285. Gemäß DIN EN 285 müssen alle Oberflächen der sterilisierten Gegenstände einem reinen, gesättigten Wasserdampf mit einer Temperatur von 134 °C über mindestens drei Minuten ausgesetzt werden. Ein dafür geeignetes Oberflächenmaterial ist beispielsweise PEEK (Polyetheretherketon).

Ein Aspekt der Nutzung eines dedizierten Gerätes ist der des Datenschutzes, da hier eine komplett gekapselte Verarbeitung der Daten unter Ausschluss jeglicher Netzzugriffe (LAN, WLAN) erfolgen kann, wenn dies erforderlich ist. Dadurch wird der Schutz sensibler Patientendaten gewährleistet. Das Gerät soll weiterhin die Sicherstellung von Hygiene und einer einfachen Einsetzbarkeit/Handhabbarkeit im klinischen Alltag unterstützen. Nicht überall kann ein PC mit Tastatur, bzw. ein Touchbasiertes Gerät (Tablet, Smartphone) eingesetzt werden. Dies hat sowohl hygienische als auch praktische Gründe. Ein für die Erkennung einer bestimmten Erkrankung dediziertes Gerät bietet den Vorteil der Kompaktheit, Bedienerfreundlichkeit, Hygiene und des Datenschutzes, da nur die für den Anwendungsfall notwendigen Ein- und Ausgabeeinheiten vorhanden sind. Ein sicheres, einfach zu reinigendes und bedienendes Gerät bietet daher wichtige und notwendige Aspekte eines Medizingerätes, weshalb ein solches auch Teil der Erfindung ist.

### Anwendungsbeispiel (hier für ein 12-Kanal EKG)

### Eingaben:

**D1:** 12-Kanal EKG (10 Sekunden) als XML-Datei:
patient123.xml
**D2:** Klinische Parameter des Patienten:

| | |
|---|---|
| Geschlecht: | weiblich |
| Alter: | 59 Jahre |
| Gewicht: | 82 kg |
| Größe: | 164 cm |
| Diabetes mellitus: | Nein |
| Bluthochdruck: | Ja |
| Erhöhtes Cholesterin: | Ja |
| Genetische Disposition von Herzerkrankungen: | Nein |
| Rauchverhalten: | Ex-Raucher |

Die Untersuchung bezieht sich auf die kardiologischen Pathologien: Narbe, Perfusionsdefizit, Entzündung und Hypertrophie. Die Untersuchung erfolgte in den Segmenten 1 bis 17 wie sie in der Abbildung Fig.1a gezeigt sind.

Die folgende Tabelle (Tabelle 2) zeigt als Ergebniswerte E die Prädiktionswerte für die verschiedenen kardiologischen Pathologien in den Segmenten 1 bis 17, wie sie nach Anwendung des erfindungsgemäßen Verfahrens mit einem neuronalen Netzwerk ermittelt werden. Dieser Prädikationswert für den Ort einer Pathologie ist ein für die Diagnose relevantes Zwischenergebnis und muss von ärztlichem Fachpersonal für eine Diagnose im medizinischen Kontext noch interpretiert werden. In diesem Anwendungsbeispiel liegt der Schwellwert für das Vorliegen einer kardiologischen Pathologie in einem bestimmten Segment bei einem Prädiktionswert von 0,5. Werte von 0,5 oder größer werden als Pathologien identifiziert. Dieser Schwellenwert wurde von ärztlichem Fachpersonal auf Grund seiner Erfahrung beispielhaft ermittelt und ist nicht Teil des erfindungsgemäßen Verfahrens.

**Tabelle 2:**

| | **Narbe** | **Perfusionsdefizit** | **Entzündung** | **Hypertrophie** |
|---|---|---|---|---|
| **Segment 1** | **0,03** | **0,66** | **0,01** | **0,04** |
| **Segment 2** | **0,02** | **0,81** | **0,10** | **0,03** |
| **Segment 3** | 0,00 | 0,00 | 0,00 | 0,00 |
| **Segment 4** | 0,00 | 0,00 | 0,00 | 0,00 |
| **Segment 5** | 0,00 | 0,00 | 0,00 | 0,00 |
| **Segment 6** | 0,00 | 0,00 | 0,00 | 0,00 |
| **Segment 7** | **0,05** | **0,77** | **0,02** | **0,06** |
| **Segment 8** | **0,01** | **0,82** | **0,02** | **0,08** |
| **Segment 9** | 0,00 | 0,00 | 0,00 | 0,00 |
| **Segment 10** | 0,00 | 0,00 | 0,00 | 0,00 |
| **Segment 11** | 0,00 | 0,00 | 0,00 | 0,00 |
| **Segment 12** | 0,00 | 0,00 | 0,00 | 0,00 |
| **Segment 13** | **0,05** | **0,75** | **0,03** | **0,07** |
| **Segment 14** | **0,01** | **0,81** | **0,01** | **0,09** |
| **Segment 15** | 0,00 | 0,00 | 0,00 | 0,00 |
| **Segment 16** | 0,00 | 0,00 | 0,00 | 0,00 |
| **Segment 17** | **0,01** | **0,81** | **0,01** | **0,10** |
| **Aggregiert (hier mit max)** | **0,05** | **0,82** | **0,10** | **0,10** |
| **Ausgabe (hier dichotom)** | **0** | **1** | **0** | **0** |

### Ergebnis:

Im dargestellten Beispiel liegt ein Perfusionsdefizit in den Segmenten 1, 2, 7, 8, 13, 14 und 17 vor. Diese Segmente sind in der Tabelle 2 fett hervorgehoben.

### Abbildungslegenden und Bezugszeichenliste

Die Abbildung 1 zeigt eine schematische Einteilung der Bereiche des menschlichen Herzens.

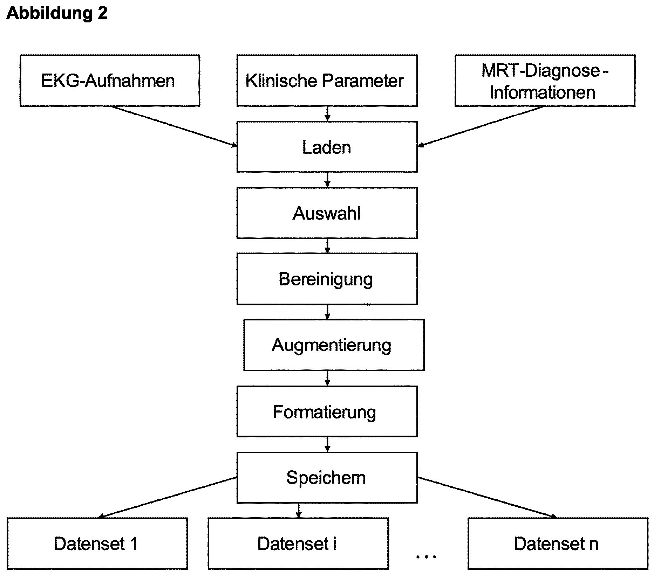

Die Abbildung 2 zeigt schematisch den Ablauf der Vorverarbeitung. Hierbei werden die MRT-Diagnose-Informationen D3 nur während der Trainingsphase aber nicht während des erfindungsgemäßen Verfahrens verwendet.

Die Abbildung 3 zeigt schematisch die erfindungsgemäße Vorrichtung 1.

### Bezugszeichen

- **1**: Vorrichtung
- **10**: Eingabeeinheit
- **20**: Auswertungseinheit
- **30**: Ausgabeeinheit
- **50**: Datenschnittstelle
- **TD1**: EKG-Trainingsmessdaten
- **TD1***: vorverarbeitete EKG-Trainingsmessdaten
- **D1**: EKG-Daten
- **D1***: vorverarbeitete EKG-Daten
- **D2**: klinische(r) Parameter
- **D2***: vorverarbeitete klinische(r) Parameter
- **D3**: MRT-Diagnose-Informationen
- **E**: Ergebnisdaten
- **N**: trainiertes Neuronales Netz

## Patentansprüche

1. Verfahren zur Vorhersage und Lokalisierung struktureller Veränderungen im Myokardgewebe, ausgewählt aus der Gruppe: Narben, Perfusionsdefizite, Entzündungen, Hypertrophien, Ischämie-bedingte Schädigungen der Zellstruktur umfassend folgende Schritte:
I. Bereitstellen der EKG-Messdaten (D1), wobei das Bereitstellen entweder über eine Eingabeeinheit (10) und/oder über eine Datenschnittstelle (50) erfolgt, wobei das Bereitstellen erst nach Abschluss der Untersuchung, nachdem die Messung am Patienten abschlossen ist, beginnt
II. Vorverarbeiten der EKG-Messdaten (D1) in vorverarbeitete EKG-Messdaten (D1*), sodass sie an ein Künstliches Neuronales Netz übergeben werden können und Übergabe der vorverarbeiteten EKG-Daten (D1*) an ein trainiertes Künstliches Neuronales Netz (N).
III. Anwendung des trainierten Künstlichen Neuronalen Netzes *N* auf die vorverarbeiteten EKG-Daten (D1*) zur Ermittlung der Ergebnisdaten (E), wobei alle Messpunkte der vorverarbeiteten EKG-Daten (D1*) verwendet werden und die Abtastfrequenz der vorverarbeiteten EKG-Daten (D1*) mindestens 50 Hz beträgt, wobei die Ergebnisdaten (E), wenigstens einen Prädiktionswert für wenigstens jede kardiale Pathologie aus der Gruppe: Narben, Perfusionsdefizite, Entzündungen, Hypertrophien für alle 17 Segmente des Myokards gemäß des 17-Segment-Modells der American Heart Association (AHA) umfassen, sodass eine Unterscheidung zwischen Narbe, Perfusionsdefizit, Entzündung und Hypertrophie getroffen wird, indem ein Prädiktionswert für all diese spezifischen strukturellen Veränderungen in allen 17 Segmenten angegeben wird, wobei die Ergebnisdaten (E) eine Vorhersage und eine Lokalisierung einer strukturellen Veränderung im Myokardgewebe ermöglichen, wobei die vollständigen vorverarbeiteten EKG-Daten (D1*) über den gesamten Verlauf einer EKG-Messung verwendet werden,
wobei das Vorverarbeiten der EKG-Messdaten (D1) und die Anwendung des trainierten Künstlichen Neuronalen Netzes N über eine Auswertungseinheit (20) erfolgt.
IV. Ausgabe der Ergebnisdaten (E), wobei die Ausgabe über eine Ausgabeeinheit (30) erfolgt.

2. Verfahren zur Vorhersage und Lokalisierung struktureller Veränderungen im Myokardgewebe gemäß Anspruch 1, **dadurch gekennzeichnet, dass** Schritt II wenigstens folgende Teilschritte zur Umwandlung der EKG-Daten (D1) in vorverarbeitete EKG-Daten (D1*) umfasst:
a. Extraktion von EKG-Daten eines einzelnen Patienten aus den EKG-Messdaten (D1)
b. Auswahl von geeigneten Datensätzen aus den EKG-Daten aus Schritt a, wobei nicht verwendbare Datensätze von der weiteren Verarbeitung ausgeschlossen werden
c. Bereinigung der Datensätze aus Schritt b, wobei diese beschnitten werden, um technische Artefakte zu beseitigen
d. Augmentierung der Datensätze aus Schritt c, wobei eindeutige Teilausschnitte aus jeder EKG-Aufnahme extrahiert werden, indem ein Fenster fixer Größe Schritt für Schritt über die Aufnahme bewegt wird, was jeweils eine neue Aufnahme generiert.
e. Formatierung der Datensätze aus Schritt d, wobei für jeden Datensatz ein Down-Sampling durchgeführt wird um hochfrequentes Rauschen und die Größe der EKG-Aufnahmen zu reduzieren
f. Speichern der vorverarbeiteten EKG-Daten (D1*) aus Schritt e

3. Verfahren zur Vorhersage und Lokalisierung struktureller Veränderungen im Myokardgewebe gemäß Anspruch 1 oder 2, weiterhin umfassend einen Schritt la, welcher vor Schritt II erfolgt und in welchem wenigstens ein klinischer Parameter (D2) empfangen wird, und weiterhin umfassend einen Schritt IIa, welcher zwischen Schritt la und Schritt III stattfindet und in welchem eine Vorverarbeitung des wenigstens einen klinischen Parameter (D2) zu einem vorverarbeiten klinischen Parameter (D2*) und eine Übergabe dieses wenigstens einen vorverarbeiteten klinischen Parameters (D2*) an ein Künstliches Neuronales Netz (N) erfolgt.

## Claims

1. Method for predicting and localising structural changes in myocardial tissue, selected from the group comprising: scarring, perfusion deficits, inflammation, hypertrophy, and ischaemia-induced damage to the cell structure, comprising the following steps:
I. Providing the ECG measurement data (D1), wherein the provision takes place either via an input unit (10) and/or via a data interface (50), wherein the provision only begins after the examination has been completed, once the measurement on the patient has been concluded
II. Pre-processing the ECG measurement data (D1) into pre-processed ECG measurement data (D1*) so that it can be transferred to an artificial neural network, and transferring the pre-processed ECG data (D1*) to a trained artificial neural network (N).
III. Application of the trained artificial neural network N to the pre-processed ECG data (D1*) to determine the result data (E), whereby all measurement points of the pre-processed ECG data (D1*) are used and the sampling frequency of the pre-processed ECG data (D1*) is at least 50 Hz, wherein the result data (E) comprise at least one prediction value for at least each cardiac pathology from the group: scarring, perfusion deficits, inflammation, hypertrophy for all 17 segments of the myocardium in accordance with the 17-segment model of the American Heart Association (AHA), such that a distinction is made between scarring, perfusion deficit, inflammation and hypertrophy is made by specifying a prediction value for all these specific structural changes in all 17 segments, wherein the result data (E) enable the prediction and localisation of a structural change in the myocardial tissue, wherein the complete pre-processed ECG data (D1*) is used over the entire course of an ECG measurement,
whereby the pre-processing of the ECG measurement data (D1) and the application of the trained artificial neural network N are carried out via an evaluation unit (20).
IV. Output of the result data (E), wherein the output is performed via an output unit (30).

2. Method for predicting and localising structural changes in myocardial tissue according to claim 1, **characterised in that** step II comprises at least the following substeps for converting the ECG data (D1) into pre-processed ECG data (D1*):
a. Extraction of ECG data for a single patient from the ECG measurement data (D1)
b. Selection of suitable data sets from the ECG data from step a, whereby unusable data sets are excluded from further processing
c. Cleaning of the data sets from step b, whereby these are trimmed to eliminate technical artefacts
d. Augmentation of the data sets from step c, whereby distinct sub-segments are extracted from each ECG recording by moving a window of fixed size step by step across the recording, which generates a new recording in each instance.
e. Formatting the data sets from step d, whereby down-sampling is performed for each data set to reduce high-frequency noise and the size of the ECG recordings
f. Saving the pre-processed ECG data (D1*) from step e

3. Method for predicting and localising structural changes in myocardial tissue according to claim 1 or 2, further comprising a step la, which takes place prior to step II and in which at least one clinical parameter (D2) is received, and further comprising a step IIa, which takes place between step la and step III and in which pre-processing of the at least one clinical parameter (D2) into a pre-processed clinical parameter (D2*) and a transfer of this at least one pre-processed clinical parameter (D2*) to an artificial neural network (N) takes place.

## Revendications

1. Procédé de prédiction et de localisation de modifications structurelles dans le tissu myocardique, choisies parmi le groupe comprenant : les cicatrices, les déficits de perfusion, les inflammations, les hypertrophies, les lésions de la structure cellulaire dues à l'ischémie, comprenant les étapes suivantes :
I. Fournir les données de mesure ECG (D1), cette fourniture s'effectuant soit via une unité d'entrée (10), soit via une interface de données (50), la fourniture ne commençant qu'une fois l'examen terminé, après que la mesure sur le patient a été achevée
II. Prétraitement des données de mesure ECG (D1) en données de mesure ECG prétraitées (D1*), de sorte qu'elles puissent être transmises à un réseau neuronal artificiel, et transmission des données ECG prétraitées (D1*) à un réseau neuronal artificiel entraîné (N).
III. Application du réseau neuronal artificiel N, après apprentissage, aux données ECG prétraitées (D1*) afin de déterminer les données de résultat (E), tous les points de mesure des données ECG prétraitées (D1*) étant utilisés et la fréquence d'échantillonnage des données ECG prétraitées (D1*) étant d'au moins 50 Hz, les données de résultat (E) comprenant au moins une valeur de prédiction pour au moins chaque pathologie cardiaque du groupe : cicatrices, déficits de perfusion, inflammations, hypertrophies pour les 17 segments du myocarde selon le modèle à 17 segments de l'American Heart Association (AHA), de sorte qu'une distinction soit faite entre cicatrice, déficit de perfusion, inflammation et hypertrophie est effectuée en indiquant une valeur prédictive pour toutes ces modifications structurelles spécifiques dans les 17 segments, les données de résultat (E) permettant une prédiction et une localisation d'une modification structurelle dans le tissu myocardique, les données ECG prétraitées complètes (D1*) sont utilisées sur toute la durée d'une mesure ECG,
le prétraitement des données de mesure ECG (D1) et l'application du réseau neuronal artificiel N entraîné s'effectuant via une unité d'évaluation (20).
IV. Sortie des données de résultat (E), la sortie s'effectuant via une unité de sortie (30).

2. Procédé de prédiction et de localisation de modifications structurelles dans le tissu myocardique selon la revendication 1, **caractérisé en ce que** l'étape II comprend au moins les sous-étapes suivantes pour la conversion des données ECG (D1) en données ECG prétraitées (D1*) :
a. Extraction des données ECG d'un patient individuel à partir des données de mesure ECG (D1)
b. Sélection d'ensembles de données appropriés parmi les données ECG de l'étape a, les ensembles de données inutilisables étant exclus du traitement ultérieur
c. Nettoyage des enregistrements de l'étape b, ceux-ci étant tronqués afin d'éliminer les artefacts techniques
d. Augmentation des enregistrements de l'étape c, des segments distincts étant extraits de chaque enregistrement ECG en déplaçant pas à pas une fenêtre de taille fixe sur l'enregistrement, ce qui génère à chaque fois un nouvel enregistrement.
e. Mise en forme des ensembles de données de l'étape d, un sous-échantillonnage étant effectué pour chaque ensemble de données afin de réduire le bruit à haute fréquence et la taille des enregistrements ECG
f. Enregistrement des données ECG prétraitées (D1*) de l'étape e

3. Procédé de prédiction et de localisation de modifications structurelles dans le tissu myocardique selon la revendication 1 ou 2, comprenant en outre une étape la, qui précède l'étape II et au cours de laquelle au moins un paramètre clinique (D2) est reçu, et comprenant en outre une étape IIa, qui a lieu entre l'étape la et l'étape III et au cours de laquelle un prétraitement dudit au moins un paramètre clinique (D2) en un paramètre clinique prétraité (D2*) et un transfert dudit au moins un paramètre clinique prétraité (D2*) vers un réseau neuronal artificiel (N) ont lieu.
